(19) Europäisches Patentamt
European Patent Office
Office européen des brevets

(11) **EP 3 351 262 A1**

(12) **EUROPEAN PATENT APPLICATION**

(43) Date of publication:
**25.07.2018 Bulletin 2018/30**

(51) Int Cl.:
*A61K 38/22* (2006.01)          *A61P 3/10* (2006.01)
*A61K 45/06* (2006.01)

(21) Application number: **17211036.3**

(22) Date of filing: **29.12.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **30.12.2016   TR 201620267**

(71) Applicant: **Istanbul Universitesi Rektorlugu 34452 Istanbul (TR)**

(72) Inventors:
• **Bolkent, Sehnaz**
  **34134 Istanbul (TR)**
• **Oktayoglu, Selda**
  **34134 Istanbul (TR)**
• **Yilmaz, Eda Oyar.**
  **Istanbul (TR)**

(74) Representative: **Mutlu, Aydin**
  **Invokat Intellectual Property Services Ltd.**
  **Kartaltepe Mh. Yildiztepe Sk.**
  **No:6-Bakirköy**
  **34145 Istanbul (TR)**

(54) **CURAGLUTIDE FOR IN TREATMENT OF PREDIABETES, DIABETES, OBESITY AND METABOLIC DISEASES ASSOCIATED THERETO**

(57)     The present invention relates to curaglutide, which is a proteolysis resistant GLP-1 analogue, for use in prophylaxis and/or treatment of pre-diabetes, diabetes, obesity and metabolic diseases associated thereto.

EP 3 351 262 A1

**SEQUENCE LISTING**

<110> Istanbul Universitesi

<120> Therapeutic Use of Curaglutide for Treatment of Prediabetes, Diabetes, Obesity and Metabolic Diseases Associated Thereto

<130> 06-17731-A

<160> 1

<170> PatentIn version 3.5

<210> 1
<211> 28
<212> PRT
<213> Homo sapiens

<400> 1

His Ala Glu Gly Thr Phe Thr Ser Asp Val Ser Ser Tyr Leu Glu Gly
1               5               10              15

Gln Ala Ala Lys Glu Phe Ile Ala Trp Leu Val Lys
            20              25

SEQUENCE LISTING

```
<110>  Istanbul Unýversitesi

<120>  Therapeutic Use of Kuraglutide for Treatment of Prediabetes,
Diabetes, Obesity and Metabolic Diseases Associated Thereto

<130>  06-17731-A

<160>  1

<170>  PatentIn version 3.5

<210>  1
<211>  28
<212>  PRT
<213>  Homo sapiens

<400>  1

His Ala Glu Gly Thr Phe Thr Ser Asp Val Ser Ser Tyr Leu Glu Gly
1                       5                           10
15

Gln Ala Ala Lys Glu Phe Ile Ala Trp Leu Val Lys
                20                          25
```

**Description**

**Technical Field of the Invention**

The present invention relates to the use of curaglutide, which is a proteolysis resistant Glp-1 analogue, for prophylaxis and treatment of the diseases caused by the cell death by autophagy, apoptosis and necrosis related lipotoxicity, glucotoxicity or glucolipotoxicity, in particular for the prophylaxis and treatment of prediabetes, diabetes, obesity and metabolic diseases associated therewith, and also to pharmaceutical compositions and combinations comprising said analogue.

**Background of the Invention**

Type 2 diabetes (T2D) is a common disease associated with serious health problems such as infection, heart attack, renal impairment, cerebral vascular diseases, blindness, gangrene in the arms and legs, and it is associated with the insulin secretion deficiency, insulin resistance, hyperglycemia and dyslipidemia from pancreatic beta cells. Lipotoxicity caused by the increase in the amount of free fatty acid causes beta cell function disorder and apoptosis. Type 2 diabetes has two significant properties. While one of them is the formation of resistance in the cells against insulin activation caused by insulin receptors and post insulin receptor disorders, the other one is a deficiency in the beta cells in the release and production of insulin. Both of them are genetically controlled factors and it is not clear yet that which one of them plays primary role. Insulin resistance causes the increase in the amount of free fatty acid in circulation and fat accumulation in the tissues except adipose tissue (ectopic). While some genes may cause insulin resistance and diabetes, epidemiological studies showed that obesity developed depending on the decrease in physical activity and the increased intake of calories cause insulin resistance and T2D. According to International Diabetes Federation (IDF), there was 336 million people with T2D in 2011 and this number increases about 4,6 million every year.

T2D occurs as a result of the increase of pancreatic beta cell mass in order to compensate the insulin deficiency felt in tissues, the disorder in glucose tolerance when beta cells cannot release sufficient insulin, and as a result, a significant increase in glucose levels in blood.

It has been known for long time that GLP-1 and its analogues that have significant effects on beta cell function, have therapeutic effect in T2D as well. GLLP-1 is an intestinal hormone and it affects glucose metabolism and nutritional behaviors, and those effects can be insulinotropic, insulinomimetic, glucagonostatic and anorectic.

US 6,268,343 B1, for instance, discloses GLP-1 and its analogues. It is stated that these analogues are disclosed for the treatment of Type 1 and Type 2 diabetes and obesity.

In WO 2005/000892 A2, it was shown that half-life and efficiency of GLP analogues is increased by adding IgG4-Fc fusion proteins to GLP-1 analogues.

WO 2007/012188 shows the compositions comprising GLP-1 fusion protein, mutant GLP 1, IgG-Fc and exendin-4. Use of these compositions for the treatment and prevention of Type I and Type II diabetes has been disclosed.

In WO 2013/060887 A1, the protective and therapeutical effect of GLP-1 analogues in heart ischemia reperfusion damage is shown.

However, since the plasma half-life of GLP-1 is short, this molecule has limited usage for the treatment of Type 2 diabetes. GLP-1 is destructed quickly by dipeptidyl peptidase IV (DPP-IV) which is a proteolytic enzyme, by way of cutting N-terminal from histidine and alanine amino acids. It is known that the half-life of GLP-1 in plasma is around 12-30 minutes.

On the other hand, as a result of the exposure of beta cells to free fatty acids in excess, it is known that cell mass and beta cell function loss relating T2D decreases. As it is stated above, beta cell disorder causes T2D.

In consideration of the prior art and the expansion rate of diabetes, effective agents are needed for the treatment and prevention of T2D, in particular, for the prevention of autophagy, apoptosis and necrosis of beta cells.

**Objects of the Invention**

The present invention aims to improve the alternative agents which can be used for the treatment and prevention of pre-diabetes, diabetes, obesity and metabolic diseases associated thereto.

The present invention also aims to improve the alternative agents which can affect autophagy, apoptosis and necrosis mechanisms.

The present invention also aims to improve the alternative agents which can be effective to prevent cell death caused by lipotoxicity, glucotoxicity or glucolipotoxicity.

A further object of the present invention is to suggest a method which involves the use of new agents in order to increase the number of beneficial pancreatic beta cells for treatment of the foregoing diseases.

## Summary of the Invention

In an aspect, the present invention relates to the use of curaglutide, in particular a pharmaceutical agent comprising the same for treatment of pre-diabetes, diabetes, obesity and metabolic diseases associated thereto.

Said pre-diabetes, diabetes, obesity and metabolic diseases associated thereto can be those diseases associated with autophagy, apoptosis and necrotic cell death originating from lipotoxicity, glucotoxicity or glucolipotoxicity. In particular, the disease herein is Type I and Type II diabetes. Said pre-diabetes can be high fasting glucose levels or impaired glucose tolerance or development of insulin resistance. On the other hand, pre-diabetes or diabetes or metabolic diseases relating obesity can be selected from the group consisting of diabetic retinopathy, diabetic maculopathy, glaucoma, retinopathy, neuropathy, nephropathy, foot ulcers, hypertension, hyperlipidemia, metabolic syndrome, gall bladder diseases, osteoarthritis, cardiovascular diseases, stroke, sleep apnea, hepatopathy, asthma, respiratory disorder, menstruation disorders, musculoskeletal disorders, dermatologic disorders, polycystic ovary syndrome and immune system disorders.

The pharmaceutical agent according to the invention can further comprise at least one additional agent selected from the group consisting of anti-obesity agents, anti-inflammatory agents, antidiabetic agents, insulin analogues, insulin sensitizer agents, insulin secretagogues, aldose reductase inhibitors, alpha glucosidose inhibitors, amylin analogues, peptide analogues, sodium glucose transporter 2 (SGLT) inhibitors, and glucosuric agents.

The antidiabetic agent herein is preferably selected from the group consisting of insulin, insulin lispro, insulin aspart, insulin glulisine, insulin zinc, isophane insulin, insulin glargine, insulin detemir, metformin, phenformin, buformin, ciglitazone, darlitazone, englitazone, lobeglitazone, netoglitazone, rivoglitazone, aleglitazar, saroglitazar, tesaglitazar, rosiglitazone, pioglitazone, troglitazone, acetohexamide, carbutamide, chlorpropamide, metahexamide, tolbutamide, tolazamide, glibenclamide, glibornuride, glicetanile, gliclazide, gliflumide, glipizide, gliquidone, glisoxepide, glyclopyramide, glimepiride, repaglinide, mitiglinide, exenatide, liraglutide, taspoglutide, albiglutide, lixisenatide, dulaglutide, semaglutide, alogliptin, anagliptin, gemigliptin, linagliptin, omarigliptin, saxagliptin, sitagliptin, teneligliptin, vildagliptin, fasiglifam, nateglinide, epalrestat, fidarestat, ranirestat, tolrestat, zenarestat, miglitol, acarbose, voglibose, pramlintide, canagliflozin, dapagliflozin, empagliflozin, remogliflozin, sergliflozin, tofogliflozin, benfluorex and bromocriptine.

The anti-obesity agent herein is preferably selected from the group consisting of 4-methyl amphetamine, amfechloral, amfepentorex, amfepramone, aminorex, amphetamine, atomoxetine, benfluorex, benzphetamine, bupropion, cathine, cathinone, chlorphentermine, ciclazindol, clobenzorex, cloforex, clominorex, clotermine, dexfenfluramine, dextroamphetamine, dexmethylphenidate, dexfenfluramine, dextroamphetamine, dexmethylphenidate, difemetorex, dimethylcathinone, diphemetoxidine, ephedrine, ephedra, ethylamphetamine, etolorex, fenbutrazate, fencamfamin, fenethylline, fenfluramine, fenproporex, fludorex, fluminorex, furfenorex, indanorex, khat, levopropylhexedrine, lisdexamfetamine, manifaxine, mazindol, mefenorex, methamphetamine, methylphenidate, norfenfluramine, pemoline, pentorex, phendimetrazine, phenethylamine, phenmetrazine, phentermin, phenylpropanolamine, picilorex, pipradrol, prolintane, propylhexedrine, pseudo-efedrine, pyrovalerone, radafaxine, reboxetine, setazindol, sibutramine, sinephrine, tesofensine, viloxazine, xylopropamine, zylofuramine, drinabant, ibipinabant, otenabant, rimonabant, rosonabant, surinabant, taranabant, 5-HTP, galactomannan, glucomannan, L-DOPA, L-phenylalanine, L-tryptophan, L-tyrosine, lorcaserin, Lu AA-33810, naltrexone, naloxone, oxyntomodulin, P57, peptide YY, topiramate, yohimbine, zonisamide, cetilistat, 2,4-dinitrophenol, dirlotapide, mitratapide, oleoyl-estrone, orlistat, simmondsin and sterculia.

The anti-inflammatory agent herein is preferably selected from the group consisting of pyrazolone/pyrazolidines, salicylates, acetic acid derivatives, oxicams, propionic acid derivatives, N-aryl anthranilic acids and coxibs. Said anti-inflammatory agent herein is more preferably selected from the group consisting of aminophenazone, ampyrone, clofenazon, famprofazone, feprazone, kebuzone, metamizole, mofebutazone, morazone, nifenazone, oxifenbutazone, phenazone, phenylbutazone, propyphenazone, sulfinpyrazone, suxibuzone, acetylsalicylic acid, aloxiprin, benorylate, carbasalate calcium, diflunisal, ethenzamide, guacetisal, magnesium salicylate, methyl salicylate, salsalate, salicylamide, salicyclic acid, sodium salicylate, aceclofenac, acemetacin, alclofenac, amphenac, bendazac, bromfenac, bumadizone, bufexamac, diclofenac, difenpramide, etodolac, felbinac, fenclozic acid, fentiazac, indometacin, indometacin farnesyl, isoxepac, ketorolac, lonazolac, acemetacin, prodolic acid, proglumetacin, sulindac, tiopinac, tolmetin, zomepirac, ampiroxicam, droxicam, lornoxicam, meloxicam, piroxicam, tenoxicam, alminoprofen, benoxaprofen, carprofen, dexibuprofen, dexketoprofen, fenbufen, fenoprofen, flunoxaprofen, flurbiprofen, ibuprofen, ibuproxam, indoprofen, ketoprofen, loxoprofen, miroprofen, naproxen, oxaprozin, pirprofen, suprofen, tarenflurbil, tepoxalin, tiaprofenic acid, vedaprofen, naproxcinod, azapropazone, etofenamate, flufenamic acid, flunixine, meclofenamic acid, mefenamic acid, morniflumate, niflumic acid, tolfenamic acid, flutiazin, apricoxib, celecoxib, cimicoxib, deracoxib, etoricoxib, firocoxib, lumiracoxib, mavacoxib, parecoxib, robenacoxib, rofecoxib, valdecoxib, aminopropionitrile, benzydamine, chondroitin sulphate, diacerein, fluproquazone, glucosamine, glucosaminoglycan, hyperforin, nabumetone, nimesulide, oxaceprol, proquazone, tenidap, and orgotein.

The pharmaceutical agent of the invention can also comprise at least one excipient. Also, at least one carrier selected from nanoparticles or liposomes can be used in order to target said pharmaceutical agent to cells or carry them by cells.

The pharmaceutical agent of the invention can be administered oral, rectal, nasal or vaginal route, or in a form suitable of intratumoral, subcutan, intracutan, intravenous, intracerebroventricular, intramuscular, intra-arterial, intratracheal or intraperitoneal, parental, or topical route. Said pharmaceutical agent is preferably in injectable form. Curaglutide dose in the pharmaceutical agent is preferably between 0.0001 µg/kg and 500 mg/kg.

In another aspect, the invention relates to an *in vitro* method for beta cell growth comprising treating of said beta cells with curaglutide. Said beta cells were found to be playing a role in the treatment of prediabetes, diabetes, obesity and the metabolic diseases associated thereto. Therefore, in another aspect, the present invention relates to the provision of pancreatic beta cells obtained by the above-mentioned method, for use in treatment of prediabetes, diabetes, obesity and metabolic diseases associated thereto.

**Brief Description of the Figures**

A brief description of the figures referred to in the context of the present invention are given hereinbelow, wherein;

Figure 1 shows the effect of palmitic acid given to INS-1 cells in different doses, on % cell viability (* $p<0.05$; ** $p<0.001$; *** $p<0.0001$).

Figure 2 shows the determination of the LC3 protein amount for the cell stimulated by palmitic acid in INS-1 cells (* $p<0.05$; *** $p<0.0001$).

Figure 3 shows the determination of cytochrome-c and active caspase-3 amount inside of the cells, in INS-1 cells (A.U. : Arbitrary Unit)(* $p<0.05$).

Figure 4 shows the effect of palmitic acid on lactate dehydrogenase enzyme release in INS-1 beta cells ($p<0.05$; ** $p<0.001$).

Figure 5 shows the effect of palmitic acid on reactive oxygen types in INS-1 beta cells (** $p<0.001$; *** $p<0.0001$).

Figure 6 shows the effect of curaglutide on cell viability in INS-1 beta cells (*$p<0.05$).

Figure 7 shows the protective and therapeutical effect of curaglutide on cell viability in INS-1 beta cells (* $p<0.05$; *** $p<0.0001$).

Figure 8 shows the protective and therapeutical effect of curaglutide on autophagy caused by palmitic acid in INS-1 beta cells (*** $p<0.0001$).

Figure 9 shows the protective and therapeutical effect of curaglutide on apoptosis caused by palmitic acid in INS-1 beta cells (* $p<0.05$; ** $p<0.001$; *** $p<0.0001$).

Figure 10 shows the protective and therapeutical effect of curaglutide on lactate dehydrogenase release stimulated by palmitic acid in INS-1 beta cells (*** $p<0.0001$).

Figure 11 shows the protective and therapeutical effect of curaglutide on ROS in INS-1 beta cells applied with palmitic acid in different doses (** $p<0.001$; ***$p<0.0001$).

**Detailed Description of the Invention**

The present invention pertains to the use of curaglutide molecule in order to prevent cell death of pancreatic beta cells caused by lipotoxicity.

In addition, the use of curaglutide molecule in order to prevent cell death of pancreatic beta cells caused by glucotoxicity and glucolipotoxicity is disclosed herein.

In this regard, an aspect of the present invention is the use of curaglutide as a medicament in prophylaxis and/or therapy of all diseases associated with apoptotic, autophagic and/or necrotic cell death caused by lipotoxicity or glucotoxicity or glucolipotoxicity.

The present invention also suggests the use of curaglutide which is a new GLP-1 analogue alone or in combination with at least one active ingredient in a suitable pharmaceutical composition, for the treatment of the diseases associated with apoptotic, autophagic and necrotic cell death.

The present invention also suggests the use of curaglutide which is a new GLP-1 analogue alone or in combination with at least one antidiabetic or antiobesity or anti-inflammatory agent, for the treatment of the diseases associated with apoptotic, autophagic and necrotic cell death. The term "cell death" as referred herein can be autophagy, necrosis and/or apoptosis.

The term "autophagy" used herein refers to a mechanism causing the macromolecules and organelles to be directed to lysosomes by being taken into a sacculation and a mechanism providing the degradation by merging with lysosome.

The term "necrosis" used herein can be defined as vacuolization of cytoplasm morphologically, loss of cell membrane integrity and swelling of organelles. The term "apoptosis" used herein refers to a mechanism taking place by the activation of cell lysis program in the removal of transformed, infected or damaged cells.

The term "GLP-1" used in the present invention can be defined as glucagon like peptide-1 and these terms can be used interchangeably.

GLP-1 is a neuroendocrine hormone, and it shows incretin effect. In other words, it causes an increase in insulin secretion when oral glucose is given.

The peptide sequence of curaglutide is Ac-HAEGTFTSDVSSYLEGQAAKEFIAWLVK-CONH2, molecular weight of which is 3126.4 Da. Curaglutide has been identified in the article by Salling, H., K. et.al., Regulatory peptides 178 (2012), 51-55, 10.1016/j.regpep.2012.06.007 DOI.

The studies conducted by the inventors which have been given in the section of Examples show that curaglutide is effective in proving of cell death of beta cells caused by lipotoxicity. In addition, the fact that curaglutide is resistant to destruction by DPP-IV provides that this agent has longer half-life and made it desirable to use therapeutically.

Said diseases relating the cell death by autophagy, apoptosis and necrosis originating from lipotoxicity or glucotoxicity or glucolipotoxicity, can be pre-diabetes, diabetes, obesity and the metabolic diseases associated thereto.

In this regard, in a preferred implementation of the invention, curaglutide molecule is used as a medicament for the prophylaxis and/or treatment of prediabetes, diabetes, obesity and the metabolic diseases associated thereto or neurodegenerative diseases.

Said diabetes mentioned herein can be Type 1 or Type 2 diabetes. Said pre-diabetes as mentioned herein can be impaired fasting glucose levels or impaired glucose tolerance or insulin resistance.

Said metabolic diseases associated with pre-diabetes or diabetes or obesity can be several of diseases such as diabetic retinopathy, diabetic maculopathy, glaucoma, nephropathy, foot ulcers, hypertension, hyperlipidemia, metabolic syndrome, gall bladder diseases, osteoarthritis, cardiovascular diseases, stroke, sleep apnea, hepatopathy, asthma, respiratory disorder, menstruation disorders, musculoskeletal disorders, dermatologic disorders, polycystic ovary syndrome and immune system disorders.

Said neurodegenerative diseases as mentioned herein can be Alzheimer's disease and other dementia types, Parkinson's disease and other diseases relating Parkinson's disease, Huntington's disease, amyotrophic lateral sclerosis (ALS), Batten's disease, motor neuron disease, spinal muscular atrophy, Prion disease, spinocerebellar ataxia, lewy body dementia, multiple sclerosis (MS), neuropathy and Friedrich's ataxia.

The present invention, in another aspect, relates to the use of curaglutide molecule in combination with at least one other antidiabetic or antiobesity or anti-inflammatory agent, for the prophylaxis and/or treatment of all of the diseases associated with cell death of pancreatic beta cells by autophagy, apoptosis and/or necrosis caused by lipotoxicity or glucotoxicity or glucolipotoxicity.

In another aspect, the present invention relates to the combined use of the pancreatic beta cells obtained with curaglutide molecule with at least one other antidiabetic or antiobesity or anti-inflammatory agent, for the prophylaxis and/or treatment of all of the diseases associated with prediabetes, diabetes, obesity or metabolic diseases associated thereto or neurodegenerative diseases of beta cells.

The antidiabetic agents which can be used in the context of the present invention can be selected from the group consisting of insulin analogues, insulin sensitizer agents, insulin secretagogues, aldose reductase inhibitors, alpha glucosidose inhibitors, amylin analogues, peptide analogues, sodium glucose transporter 2 (SGLT) inhibitors, and glucosuric agents.

The insulin which can be used in a combination with curaglutide in the context of the present invention can be selected from the group consisting of insulin, insulin lispro, insulin aspart, insulin glulisine, insulin zinc, isophane insulin, insulin glargine, and insulin detemir.

The insulin sensitizer agents which can be used in a combination with curaglutide in the context of the present invention can be selected from the group consisting of metformin, phenformin, buformin, ciglitazone, darlitazone, englitazone, lobeglitazone, netoglitazone, rivoglitazone, aleglitazar, saroglitazar, tesaglitazar, rosiglitazone, pioglitazone, and troglitazone.

The insulin secretagogues which can be used in a combination with curaglutide in the scope of the present invention can be selected from the group consisting of acetohexamide, carbutamide, chlorpropamide, metahexamide, tolbutamide, tolazamide, glibenclamide, glibornuride, glicetanile, gliclazide, gliflumide, glipizide, gliquidone, glisoxepide, glyclopyramide, glimepiride, repaglinide, mitiglinide, exenatide, liraglutide, taspoglutide, albiglutide, lixisenatide, dulaglutide, semaglutide, alogliptin, anagliptin, gemigliptin, linagliptin, omarigliptin, saxagliptin, sitagliptin, tenegliptin, vildagliptin, fasiglifam, and nateglinide.

The aldose reductase inhibitors which can be used in a combination with curaglutide within the scope of the present invention can be selected from the group consisting of epalrestat, fidarestat, ranirestat, tolrestat, and zenarestat.

The alpha glucosidose inhibitors which can be used in a combination with curaglutide within the scope of the present invention can be selected from the group consisting of miglitol, acarbose, and voglibose.

The amylin analogue which can be used in a combination with curaglutide within the scope of the present invention is pramlintide.

The sodium glucose transporter 2 (SGLT) inhibitors which can be used in a combination with curaglutide within the scope of the present invention can be selected from the group consisting of canaglifozin, dapagliflozin, empagliflozin, remogliflozin, sergliflozin, and tofogliflozin.

In addition to the ones given above, the antidiabetic agent can be benfluorex or bromocriptine.

Said antiobesity agents can be selected from the group consisting of amphetamine, amfecloral, amfepentorex, amfepramone, aminorex, amphetamine, atomoxetine, benfluorex, benzphetamine, bupropion, cathine, cathinone, chlorphentermine, ciclazindol, clobenzorex, cloforex, clominorex, clotermine, dexfenfluramine, dextroamphetamine, dexmethylphenidate, dexfenfluramine, dextroamphetamine, dexmethylphenidate, difemetorex, dimethylcathinone, diphemetoxidine, ephedrine, ephedra, ethylamphetamine, etolorex, fenbutrazate, fencamfamin, fenethylline, fenfluramine, fenproporex, fludorex, fluminorex, furfenorex, indanorex, khat, levopropylhexedrine, lisdexamfetamine, manifaxine, mazindol, mefenorex, methamphetamine, methylphenidate, norfenfluramine, pemoline, pentorex, phendimetrazine, phenethylamine, phenmetrazine, phentermin, phenylpropanolamine, picilorex, pipradrol, prolintane, propylhexedrine, pseudo-efedrine, pyrovalerone, radafaxine, reboxetine, setazindol, sibutramine, synephrine, tesofensine, viloxazine, xylopropamine, zylofuramine, drinabant, ibipinabant, otenabant, rimonabant, rosonabant, surinabant, taranabant, 5-HTP, galactomannan, glucomannan, L-DOPA, L-phenylalanine, L-tryptophan, L-tyrosine, lorcaserin, Lu AA-33810, naltrexone, naloxone, oxyntomodulin, P57, peptide YY, topiramate, yohimbine, zonisamide, cetilistat, 2,4-dinitrophenol, dirlotapide, mitratapide, oleoyl estrone, orlistat, simmondsin and sterculia.

The anti-inflammatory agents which can be used in a combination with curaglutide within the scope of the present invention can be selected from the group consisting of pyrazolone/pyrazolidines, salicylates, acetic acid derivatives, oxicams, propionic acid derivatives, N-aryl anthranilic acids, coxibs and general groups consisting of other agents useful for this purpose.

The pyrazolone/pyrazolidines which can be used in a combination with curaglutide in the context of the present invention can be selected from the group consisting of aminophenazone, ampyrone, clofenazon, famprofazone, feprazone, kebuzone, metamizole, mofebutazone, morazone, nifenazone, oxifenbutazone, phenazone, phenylbutazone, propyphenazone, sulfinpyrazone, and suxibuzone.

The salicylates which can be used in a combination with curaglutide in the context of the present invention can be selected from the group consisting of acetylsalicylic acid, aloxiprin, benorylate, carbasalate calcium, diflunisal, ethenzamide, guacetisal, magnesium salicylate, methyl salicylate, salsalate, salicylamide, salicyclic acid, and sodium salicylate.

The acetic acid derivatives which can be used in a combination with curaglutide in the context of the present invention can be selected from the group consisting of aceclofenac, acemetacin, alclofenac, amphenac, bendazac, bromfenac, bumadizone, bufexamac, diclofenac, difenpramide, etodolac, felbinac, fenclozic acid, fentiazac, indometacin, indometacin farnesyl, isoxepac, ketorolac, lonazolac, acemetacin, prodolic acid, proglumetacin, sulindac, tiopinac, tolmetin, and zomepirac.

The oxicams which can be used in a combination with curaglutide in the context of the present invention can be selected from the group consisting of ampiroxicam, droxicam, lornoxicam, meloxicam, piroxicam, and tenoxicam.

The propionic acid derivatives which can be used in a combination with curaglutide in the context of the present invention can be selected from the group consisting of alminoprofen, benoxaprofen, carprofen, dexibuprofen, dexketoprofen, fenbufen, fenoprofen, flunoxaprofen, flurbiprofen, ibuprofen, ibuproxam, indoprofen, ketoprofen, loxoprofen, miroprofen, naproxen, oxaprozin, pirprofen, suprofen, tarenflurbil, tepoxalin, tiaprofenic acid, vedaprofen, and naproxcinod.

The N-aryl anthranilic acids which can be used in a combination with curaglutide in the context of the present invention can be selected from the group consisting of azapropazone, etofenamate, flufenamic acid, flunixine, meclofenamic acid, mefenamic acid, morniflumate, niflumic acid, tolfenamic acid, and flutiazin.

The coxibs which can be used in a combination with curaglutide in the context of the present invention can be selected from the group consisting of apricoxib, celecoxib, cimicoxib, deracoxib, etoricoxib, firocoxib, lumiracoxib, mavacoxib, parecoxib, robenacoxib, rofecoxib, and valdecoxib.

The other agents which can be used in a combination with curaglutide in the scope of the present invention can be selected from the group consisting of aminopropionitrile, benzydamine, chondroitin sulphate, diacerein, fluproquazone, glucosamine, glucosaminoglycan, hyperforin, nabumetone, nimesulide, oxaceprol, proquazone, tenidap, and orgotein.

While the usage of curaglutide in combination with antidiabetic, antiobesity, and anti-inflammatory drugs is mentioned, the usage in combination covers also the drugs that are not present in the foregoing lists and covers also the drugs already synthesized or to be synthesized which are in an improvement stage.

In another aspect, the present invention relates to the pharmaceutical formulations comprising curaglutide. Said pharmaceutical formulations may comprise at least one excipient.

The pharmaceutical formulations according to the present invention may comprise at least one active ingredient in addition to curaglutide. The other active ingredient can be selected from antidiabetic, antiobesity or anti-inflammatory agents explained above in detail or from the combinations thereof.

As the other active ingredient can be formulated along with curaglutide, it can also be formulated separately from curaglutide and it can be administrated to the patient together, intermittently or sequentially in different times.

The formulations according to the present invention can be prepared in any dosage form known in the art. Said dosage form can be administrated to a patient by way of oral, rectal, nasal or vaginal routes, or intratumoral, subcutan, intracutan, intravenous, intracerebroventricular, intramuscular, intra-arterial, intratracheal, intraperitoneal, parental, or topical routes, or by way of application via medical devices.

The formulations according to the invention can be administrated to patient via nanoparticles, liposomes and similar carriers, and can be formulated by being targeted to the cells or suitably to be carried by cells or can be administrated to the patients by these ways.

The formulations according to the invention can be formulated in a suitable form to be administrated to patient by way of nasal, oral, aerosol, rectal or vaginal formulations, and they can be administrated to the patients by these ways.

The pharmaceutical compositions according to the invention can be administrated parentally in an injectable formulation form. The injectable formulations can be formulated by using a sterile solvent or a pharmaceutically acceptable carrier. The pharmaceutically acceptable carrier can be selected, although not limited, from sterile water, physiological saline solution or cell culture mediums present in the art.

The pharmaceutical compositions according to the present invention can be used for the prophylaxis and/or treatment of the diseases associated with autophagic, necrotic and apoptotic cell death by lipotoxicity, glucotoxicity, glucolipotoxicity, and preferably for the treatment of prediabetes, diabetes, obesity or metabolic diseases associated thereto or neurodegenerative diseases. Curaglutide molecule can be used in a dose between 0.0001 microgram/kilogram ($\mu$g/kg) and 500 milligram/kilogram (mg/kg) for the prophylaxis and/or treatment of the diseases associated with autophagic, necrotic and apoptotic cell death by lipotoxicity, glucotoxicity, glucolipotoxicity, and preferably for the treatment of prediabetes, diabetes, obesity or metabolic diseases associated thereto or neurodegenerative diseases.

The features described according to the present invention can be combined with one another if required.

The examples showing the working principles of the invention and giving detailed information relating the invention are given hereinbelow. The examples are given in order to provide better understanding of the invention; however, the scope of the invention is not limited by these examples.

**EXAMPLES**

**Example 1: Materials and Preparation Methods**

*A) Cell Culture*

INS-1 cell strain from rat origin used in the studies was proliferated by performing routine passages once per week. The cells were grown under 1 atm pressure, in the incubator containing 5% $CO_2$ and 95% air, in powder RPMI 1640 medium containing heat inactivated 10% fetal bovine serum (FBS) and antibiotics (100 unit/ml penicillin G100, 100 $\mu$g/ml streptomycin), 1% sodium pyruvate, hepes, sodium bicarbonate and beta-mercaptoethanol at 37 °C. The cells produced in 75 cm$^2$ sterile culture plate were used for the experiment when they covered 60-80% of the culture plate.

The cells were frozen in order to be stored for long time. For this, the cells were first frozen by preparing the freezing medium in different combinations and a week later, several vials were opened from the combinations, cell viability was determined, the freezing medium containing 10% dimethyl sulphoxide (DMO) and 90% fetal bovine serum (FBS) was chosen. For this operation, grown cells were removed by trypsin, total cell amount in Thoma microscope slide was calculated. Then, the cells were collected by centrifuging in 1500 rpm for 5 minutes. Cell pellet was suspended in freezing medium in a way such that it has a density of 1-1.5 million cells/ml and transferred into cryotubes. The cryotubes were kept at -86 °C overnight, and then they were taken next day in order to store at -152 °C for long time.

When the frozen cells were needed to be opened, the crypto tubes were heated rapidly up to 37 °C and the cell suspension were transferred into the centrifuge tube containing 5 ml medium, and then the cells were centrifuged in 1500 rpm for 5 minutes. The cell pellet obtained was washed again in 5 ml medium in order to remove DMSO successfully and centrifuged again. This second cell pellet was suspended in small amount of medium and transferred into 25 cm$^2$ flasks.

B) Dissolution and Dilution of Palmitic Acid

10.26 mg palmitic acid was weighed, 500 $\mu$l methanol was added, and it was left in order to dissolve overnight by mixing in water bath at 60 °C. Then it was filtered by completing it to 10 ml with RPMI 1640 containing 0.5% bovine serum albumin (BSA), but not containing FBS. It was used by diluting according to the concentrations to be used.

C) Dissolution and Dilution of Curaglutide

Peptide with at least 95% purity was obtained from the firm Invitrogen and it was dissolved according to the usage specification. 400 $\mu$l acetonitrile was added to 5.4 mg peptide and vortexed well in order to dissolve, and then it was dissolved by adding 400 $\mu$l ultra-pure water. Then it was added to centrifuge tube containing 9200 $\mu$l PBS (pH 7.4) by continuously mixing in vortex. The obtained peptide solution was aliquoted, a part of which was transferred to -86 °C, while a small part of it was transferred to -20 °C. It was applied to the cells by filtering from a membrane having the pore diameter of 0.22 $\mu$m, after preparing in suitable concentrations, when it was used.

**Example 2: Dose and Time Defining Experiments for Palmitic Acid**

The cells to be used in the experiments were removed from the surface of culture plate where they stuck, when they cover 60-80% of the culture plate, by using trypsin. In order to prevent the effect of trypsin to cells whose removal was monitored under microscope, the medium with the equal amount as trypsin was added and the cell suspension was collected in the centrifuge tube and centrifuged in 1500 rpm for 5 minutes. The supernatant was removed after centrifuge, the medium was added to the cells remained in the bottom part of the tube and pipetted in order to form single cell suspension. The total amount of cells was calculated by using hemocytometer. 50 000 INS-1 beta cells taken from 100% viable single cell suspension, whose cell amount was calculated, were cultured to each of the culture plates with 96 wells. The cultured cells were incubated at 37 °C for 24 hours in order to provide the sticking to the surface of culture plate and growing. After 24 of incubation, the cells were treated with 0.25 mM, 0.5 mM, 0.75 mM, 1 mM, 1.25 mM, 1.5 mM, 1.75 mM, 2 mM, 2.5 mM, 2.75 mM, 3 mM, 3.25 mM, 3.5 mM, 3.75 mM, 4 mM, 4.25 mM, 4.5 mM, 4.75 mM, 5 mM palmitic acid which were the chosen doses for palmitic acid. 10 wells including the control group were used in the experiments and 24-hour experiment groups were formed for each of the concentrations.

**Example 3: Dose and Time Determining Experiments for Curaglutide**

After the removal of the grown cells by trypsin, culturing was performed to 96 wells in a way such that there would be 50 000 cells in 200 $\mu$l. The cultured cells were incubated at 37 °C for 24 hours in order to provide growing. After 24 h of incubation, the cells were treated with 1nM, 10nM, 100nM curaglutide which were the chosen doses. 10 wells including the control group were used in the experiments and 24-hour experiment groups have been formed for each of the concentrations.

**Example 4: Determination of Cell Viability by MTT Test**

MTT ([3-(4, 5-dimethyl tiazol-2-il)-2, 5 diphenyl tetrazolium bromide]) test is a drug sensitivity test based on cell culture principal, it aims to assess the cell growth and/or cell death in an indirect way. This frequently used test differs from the other testing methods, with the advantages that it is fast, sensitive, reproducible and easy to apply. The cracking of the tetrazolium ring of MTT paint by mitochondria in living cells underlies the basis of this method. This reaction is catalyzed by succinate dehydrogenase which is a mitochondrial enzyme. MTT is absorbed to cells actively and it is reduced to formazan which is non-soluble in water and has blue-purple color, by the reaction taking place in mitochondria. The MTT reducing properties of the cells are accepted as the cell viability criteria and they are directly proportionate to the paint density and living cell amount obtained by MTT analyze. While living cells with non-damaged mitochondria function are painted with purple color, cells that are dead or with damaged mitochondria function are not painted. Following bringing the formazan crystals formed by incubated living cells into soluble form, reaction product is read calorimetrically.

A) Preparation of MTT Solution

50 mg MTT (AppliChem A2231 0001) was weighed, it was dissolved by completing 10 ml with RPMI 1640 medium via vortex, with paying attention that there are no not-dissolved paint particles left, and they were obtained by filtering through 0.22 $\mu$m filter. It was used by preparing a fresh one each time.

B) Treating of Cells with MTT

The cells treated with toxic or protective agent were used in the experiments as control group. MTT solution having 20 $\mu$l/well volume, was added to the cells cultured to the culture plates having 96 wells and treated with predetermined dose and time of peptide and fatty acid, after targeted incubation time. Following addition of MTT solution, the culture plates were held by covering with aluminum foil in stove containing humid air with 5% $CO_2$, at 37 °C for 2 hours. At the end of targeted waiting period, it was detected by using phase contrast microscope that purple colored formazan crystals were formed in living cells, in spite of that dead cells were not painted. In order to bring formazan crystals into soluble form, solvent containing paint was removed from the wells and 75 $\mu$l isopropanol was added to each well. Following the addition of isopropanol, culture plate, where dissolution of formazan crystals was observed, was shaken in 250 rpm for 2 minutes by putting them on orbital shaker. After shaking, culture plate was transferred into ELISA plate reader and the values were read in 570-630 nm wavelengths. Viability ratio in control and experiment groups were examined by comparing with absorbance values.

**Example 5: Measurement of Lactate Dehydrogenase Release**

Lactate dehydrogenase (LDH) release test is based on the measurement of the activity of lactate dehydrogenase which is a cytoplasmic enzyme released from the dead cells or cells with damaged plasma membrane and it is used in cellular cytotoxicity assessment. The cytotoxic effect of palmitic acid on mice pancreatic INS-1 cell strain was determined by LDH activity released from the damaged cells and for this purpose Cytotoxicity Determination Kit, LDH (Roche Molecular Marker) was used.

In LDH test, as well as the experimental groups wherein palmitic acid was applied, negative control group for spontaneous LDH release from the cells and positive control group for LDH release were studied. Since palmitic acid in 0.5, 0.75 and 1 mM doses decreases the cell viability, these doses and the control group of the highest dose were studied. The cells were removed by trypsin-EDTA and centrifuged, after growth in 75 cm$^2$ flask. The cells were suspended in medium in a way such that there would be 50 000 cells in 100 $\mu$l, and 100 $\mu$l cell suspension was transferred into plates with 96 wells. 10 wells were studied for each of the control and experiment groups and the experiment was repeated for 3 times. After the incubation for 24 hours, the control groups were washed with PBS, and they were freshened with the medium without sodium pyruvate and beta-mercaptoethanol. The medium of the experiment groups was changed with the medium containing different concentrations of palmitic acid. Following the incubation of the cells with palmitic acid for 24 hours, LDH activity released from the cells was determined according to the instructions of the manufacturer. 5 $\mu$l lysis solution (2% Triton-X-100) was added to the wells of positive control group and the plate was incubated at 37 °C for 15 minutes. In order to measure LDH activity, the fresh reaction mixture prepared containing catalyst (Diaphorase+NAD mixture) and paint solution (Iodotetrazolium chloride (INT) and sodium lactate) was added to each of the wells. Following the incubation at room temperature for 30 minutes, the reaction was stopped by adding 50 $\mu$l stopping solution (IN HCl) to each of the wells. The plates were shaken in an orbital shaker for 10 seconds and the spectrophotometric absorbance was measured in 490 nm test wavelength and 690 nm reference wavelength. The results were determined as "cytotoxicity %". The average absorbance values of negative control were extracted from control and experiment groups and they were calculated by using the formula below by comparing control and experiment groups according to positive control group, and by assuming that positive control group is 100% cytotoxic:

$$100\% \ \text{Cytotoxicity} = \frac{(Experiment \ or \ Control \ Value) - (Negative \ Control)}{(Positive \ Control) - (Negative \ Control)} \times 100$$

**Example 6: ROS Measurement**

2',7'-dichlorodihydrofluorescein diacetate (H2DCFDA) is a chemically reduced form of fluorescein which is used as a marker in cells for ROS. When this compound oxidizes by oxidants or free radicals, fluorescent dichlorofluorescein (DCF) will occur.

INS-beta cells were cultured into the plates covered with black with 96 wells in a way such that there would be 50 000 cells in 100 $\mu$l. After the growth of the cells, the experiment group (only palmitic acid given) and the groups wherein curaglutide as both protective and therapeutical against palmitic acid was given were removed at the end of the experiment, and each of the wells were washed with PBS. Then 100 $\mu$l PBS was added to every well. Then, start of experiment was accepted as 0$^{th}$ hour by adding 10 mM DCFA and the measurements were taken for 3 hours in every 30 minutes, including 0$^{th}$ hour. The absorbance values of each group and the absorbance values in 3$^{rd}$ hour was calculated by assuming the value of the control group as 100%.

### Example 7: Obtaining Lysate

Trypsin is applied to 75 cm$^2$ flask containing INS-1 mice beta cells and it is centrifuged in 1500 rpm for 15 minutes. According to the density of pellet formed after centrifuge, it is centrifuged at 4 °C in 1500 rpm for 5 minutes by providing the washing with pipetting with PBS and transferring into 2 ml eppendorf. The supernatant was removed, lysis buffer was added according to the density of pellet and pipetting is performed carefully. It was left for 30 minutes on the ice by vortexing in every 10 minutes. Then the cells were allowed to lysis by ultrasonication for 10 seconds for 5 times, keeping on ice for 10 seconds and they were centrifuged at 4 °C in 13000 rpm for 10 minutes. The supernatant occurred was taken to 0,2 ml eppendorf tubes, and they were taken to -86 °C temperature. These operations were repeated for 3 times for each of the control and experiment groups.

### Example 8: Bradford Protein Quantitation

Bradford method was used in order to determine the protein content. First, a standard curve was formed by using BSA (bovine serum albumin) having a concentration of 1.46 mg/ml. This curve was prepared as following: Bradford solution was added into 80 μg/ml, 40 μg/ml, 20 μg/ml, 10 μg/ml BSA, their absorbance was measured in 595 nm, it was diluted in a way that the lysates are 1/100 and 160 μl was taken, their absorbance in the same wavelengths was determined. After absorbance/concentration graph of the standards was obtained, the concentrations of the samples whose concentrations are known were determined by using the slope equation of this graph.

### Example 9: Western Blotting

Protein bands separated by electrophoresis in gel were transferred from the gel into nitrocellulose membrane. After membrane transferring operation, transfer efficiency was controlled for 15 minutes by using Ponceau S painting solution.

The membrane wherein the proteins were transferred, was incubated at room temperature for 1 hour with blocking solution prepared with skimmed milk powder. The membranes were incubated with primary antibodies suitable diluting ratios (Table 1) for 1 night at +4 °C, following the blocking operation. Following this operation, washed membranes were treated with peroxidase marked goat anti-rabbit secondary IgGs (Santa Cruz, CA, USA, sc-2004) at room temperature for 1 hour. Following the treatment with secondary antibodies of membranes, they were washed with washing solution and shaken with pure water. Then, the specific protein bands were monitored by KodakGL1500 gel monitoring system, by chemiluminescence method using Western Blotting Luminol Reagent (Santa Cruz, CA, USA, sc-2048) chemiluminescence substrate. Their densitometric analysis were performed by "Kodak Molecular Imaging Systems" software. The statistical analysis between the bands were performed by normalizing the band densities of the protein samples whose densitometric analysis were performed, to the density of β-actin protein.

**Table 1:** The primary antibodies used in Western blotting method, the dilution ratios and periods thereof

| Primary Antibodies | Dilution Ratio | Incubation Period and Condition |
|---|---|---|
| Rabbit anti-p53 | 1:1000 | All night, +4 °C |
| Rabbit anti-cytochrome-c | 1:1000 | All night, +4 °C |
| Rabbit anti-caspase-3 | 1:1000 | All night, +4 °C |
| Rabbit anti-LC3 | 1:1000 | All night, +4 °C |
| Rabbit anti-β-actin | 1:500 | All night, +4 °C |

### Example 10: Cell Viability Test Findings of Palmitic Acid

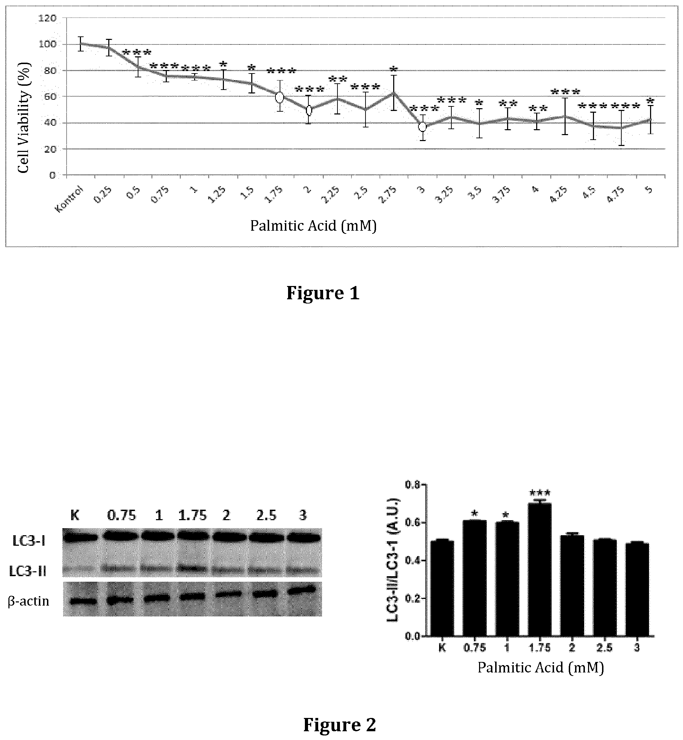

Different doses of palmitic acid were applied to INS-1 cells. The effect of palmitic acid given in wide range as mM on cell viability is shown in Figure 1, by determining with MTT technique.

The cell viability values (%) were found as 100±5.20% in control group; 92±2.40% in 0.25 mM palmitic acid group; 81.5±7.60% in 0.5 mM palmitic acid group; 75.63±4.37% in 0.75 mM palmitic acid group; 74.80±2.35% in 1 mM palmitic acid group; 72.7±7.7% in 1.25 mM palmitic acid group; 69.9±7.40% in 1.5 mM palmitic acid group; 60.6±11.60% in 1.75 mM palmitic acid group; 50.1±11% in 2 mM palmitic acid group; 58.4±11.5% in 2.25 mM palmic

acid group; 49.9±3% in 2.5 mM palmitic acid group; 62.9±4.3% in 2.75 mM palmitic acid group; 36.5±3.3% in 3 mM palmitic acid group; 44.14±8.7% in 3.25 mM palmitic acid group; 39.4±11.40% in 3.5 mM palmitic acid group; 42.9±8.2% in 3.75 mM palmitic acid group; 41.04±6.6% in 4 mM palmitic acid group; 44.94±11.8% in 4.25 mM palmitic acid group; 37.3±10.5% in 4.5 mM palmitic acid group; 35.7±3.7% in 4.75 mM palmitic acid group; 42.14±4.4% in 5 mM palmitic acid group.

This decrease in cell viability in all of the doses according to control group occurred in a statistically meaningful level (Figure 1). According to the results of the repetitive experiments performed, the application of 0.25 mM palmitic acid provided that the cells stay alive, while the application of 2 mM palmitic acid provided cell death by 50%, and the cell death decreased under 50% after the application of 3 mM palmitic acid. Therefore, in order to determine the type of cell death in toxicity created by palmitic acid, it was decided that the research will continue in the doses 0.75, 1, 1.75, 2, 2.5 and 3 mM, where the cell viability decreases prominently.

**Example 11: Determination of the Effect of Palmitic Acid Application on Autophagy**

In order to determine the autophagic dose, LC3-II protein amounts which are the autophagic markers were determined by western blotting method by obtaining lysate from the cells on which 0.75, 1, 1.75, 2, 2.5 and 3 mM palmitic acid was applied. The graph obtained from western blotting bands and the calculation performed as a result of the analysis of these bands is shown in Figure 2.

According to the results, LC3-II amounts in INS-1 cells were found as 0.50±0.01 for the control group; 0.60±0.00 for the 0.75 mM palmitic acid group; 0.60±0.00 for the 1 mM palmitic acid group; 0.70±0.03 for the 1.75 mM palmitic acid group; 0.52±0.02 for the 2 mM palmitic acid group; 0.50±0.00 for the 2.5 mM palmitic acid group; 0.49±0.01 for the 3 mM palmitic acid group. A statistically significant result was obtained when the groups were compared with each other (Figure 2). As a result of the binary comparisons, LC-3 II amount showed a statistically significant increase in comparison with the control group, at the doses wherein 0.75, 1, 1.75 mM palmitic acid was given ($p < 0,05$; $p < 0,001$; $p < 0,0001$). Since the highest statistical increase was observed in the group wherein 1.75 mM palmitic acid was given, this dose was determined as the dose stimulating autophagy in INS-1 beta cells.

**Example 12: Determination of the Effect of Palmitic Acid Application on Apoptosis in INS-1 Cells**

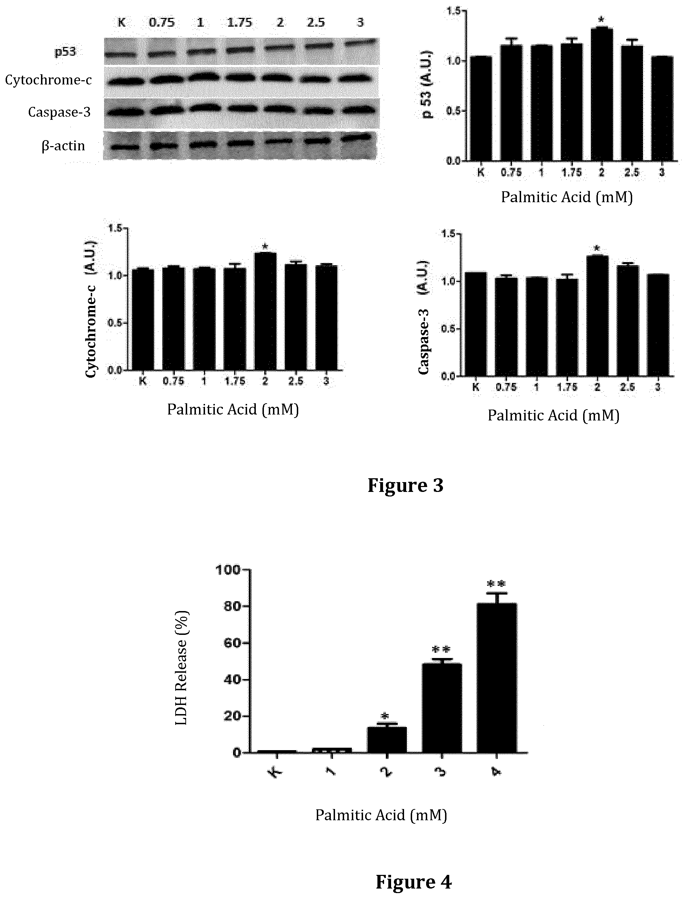

In order to determine the apoptosis dose, p 53, cytochrome-c and active caspase-3 protein amounts were determined by western blotting method by obtaining lysate from the cells in which 0.75, 1,1.75,2,2.5 and 3 mM palmitic acid were applied. The graph obtained from western blotting bands and the calculation performed as a result of the analysis of these bands is shown in Figure 3.

According to the results, p-53 amounts in INS-1 cells were found as 1.04±0.00 for the control group; 1.1±0.00 for the 0.75 mM palmitic acid group; 1.14±0.00 for the 1 mM palmitic acid group; 1.16±0.05 for the 1.75 mM palmitic acid group; 1.3±0.02 for the 2 mM palmitic acid group; 1.14±0.06 for the 2.5 mM palmitic acid group; 1.03±0.00 for the 3 mM palmitic acid group. A statistically significant result among 2 and 3 mM was obtained when the groups were compared with each other ($p < 0,05$). As a result of the binary comparisons, p 53 amount showed a statistically significant increase in comparison with the control group, at doses where 2 mM palmitic acid was given ($p < 0,05$).

According to the results, cytochrome-c amounts in INS-1 cells were found as 1.05±0.02 for the control group; 1.07±0.02 for the 0.75 mM palmitic acid group; 1.06±0.01 for the 1 mM palmitic acid group; 1.07±0.05 for the 1.75 mM palmitic acid group; 1.23±0.00 for the 2 mM palmitic acid group; 1.11±0.04 for the 2.5 mM palmitic acid group; 1.09±0.02 for the 3 mM palmitic acid group. A statistically significant result among 1.75 and 2 mM was observed when the groups were compared with each other ($p < 0,05$). As a result of the binary comparisons, cytochrome-c amount showed a statistically significant increase in comparison with the control group, at doses where 2 mM palmitic acid was given ($p < 0,05$).

According to the results, active caspase-3 amounts in INS-1 cells were found as 1.08±0.00 for the control group; 1.03±0.03 for the 0.75 mM palmitic acid group; 1.03±0.00 for the 1 mM palmitic acid group; 1.02±0.05 for the 1.75 mM palmitic acid group; 1.26±0.00 for the 2 mM palmitic acid group; 1.15±0.03 for the 2.5 mM palmitic acid group; 1.06±0.00 for the 3 mM palmitic acid group. A statistically significant result among 1.75 and 2 mM was obtained when the groups were compared with each other ($p < 0,05$). As a result of the binary comparisons, active caspase-3 amount showed a statistically significant increase in comparison with the control group, at doses where 2 mM palmitic acid was given ($p < 0,05$).

p 53, cytochrome-c and active caspase-3 apoptotic protein amounts were found to be high compatibly with each other at the dose of 2 mM and this dose was accepted as the palmitic acid dose which induces apoptosis in INS-1 beta cells.

**Example 13: Determination of The Effect of Palmitic Acid Application on Necrosis in INS-1 Cells**

Lactate dehydrogenase release in INS-1 cells wherein different palmitic acid doses were applied was measured using cytotoxicity kit and the results are shown in Figure 4.

According to the results, cytotoxicity value percentages in INS-1 cells were found as $0 \pm 0\%$ for the control group; $1 \pm 0\%$ for the 1 mM palmitic acid group; $13.5 \pm 2.4\%$ for the 2 mM palmitic acid group; $48.2 \pm 2\%$ for the 3 mM palmitic acid group; $81.3 \pm 5.9\%$ for the 4 mM palmitic acid group. A statistically significant result among doses was obtained when the groups were compared with each other ($p<0,05$; $p<0,001$). As a result of the binary comparisons, LDH amount showed a statistically significant increase in comparison with the control group, at the doses of 3 and 4 mM ($p<0,001$). The necrotic dose was chosen as 3 and 4 mM since palmitic acid in 4 mM dose exhibits very high toxic effect.

**Example 14: Determination of the Effect of Palmitic Acid Application on the Production of Reactive Oxygen Derivatives**

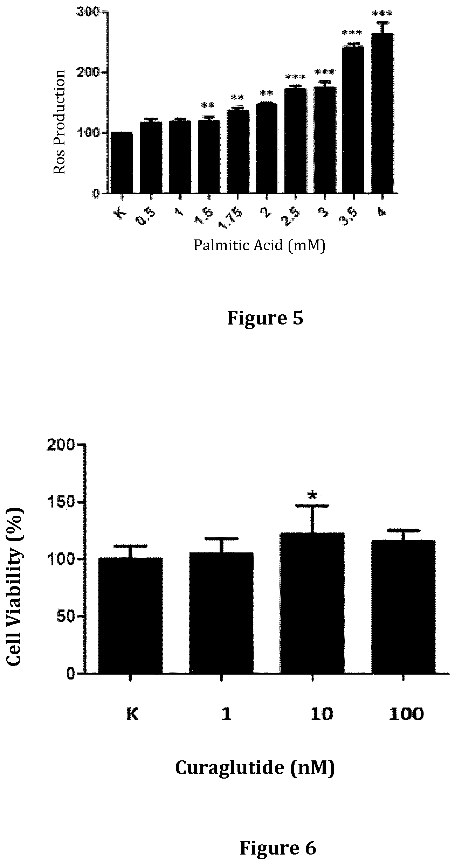

ROS measurement was performed by applying H 2 DCFDA occurred as a result of chemical reduction of fluorescein which is used as a marker for reactive oxygen derivatives (ROS) to INS-1 beta cells wherein palmitic acid was applied in different doses, and the results are shown in Figure 5.

According to the results, ROS measurement percentages in INS-1 cells were found as $100 \pm 7.02\%$ for the control group; $116 \pm 6.01\%$ for the 0.5 mM palmitic acid group; $117 \pm 2.5\%$ for the 1 mM palmitic acid group; $119.7 \pm 7.0\%$ for the 1.5 mM palmitic acid group; $136.5 \pm 5.6\%$ for the 1.75 mM palmitic acid group; $145.8 \pm 3.8\%$ for the 2 mM palmitic acid group; $172.1 \pm 6.1\%$ for the 2.5 mM palmitic acid group; $174.8 \pm 9.9\%$ for the 3 mM palmitic acid group; $241.6 \pm 5.5\%$ for the 3.5 mM palmitic acid group; $262.2 \pm 9.9\%$ for the 4 mM palmitic acid group. As a result of the binary comparisons, a significant increase was observed in the groups of 1.5, 1.75, 2, 2.5, 3, 3.5 and 4 mM palmitic acid as compared to the control group ($p<0,001$; $p<0,0001$).

**Example 15: Cell Viability Test Findings of Curaglutide**

Curaglutide was incubated for 24 hours in different concentrations (1, 10 and 100 nM) in order to observe its effects on INS-1 mice pancreatic cell strain viability. The effect of curaglutide which is given in different doses as nM on cell viability is shown in Figure 6.

According to the results, cell viability values were found as $100 \pm 3.3\%$ in the control group; $104.50 \pm 3.70\%$ in the 1 nM group; $121.80 \pm 7.30\%$ in the 10 nM group; $115.20 \pm 3.30\%$ in the 100 nM group. Statistically significant results among doses were obtained when the groups are compared with each other ($p<0,05$). As a result of the binary comparisons, a significant increase in cell viability in 10 nM was observed as compared to the control group ($p<0,05$).

**Example 16: Findings for Cell Viability Assay in the Condition where Curaglutide and Palmitic Acid have been Applied Together**

Firstly, curaglutide was taken under consideration with respect to both therapeutic and protective effects against cytotoxicity occurred by palmitic acid in INS-1 rat pancreatic beta cell lineage.

A) Protective and Therapeutic Effect of Curaglutide on INS-1 Beta Cell Death

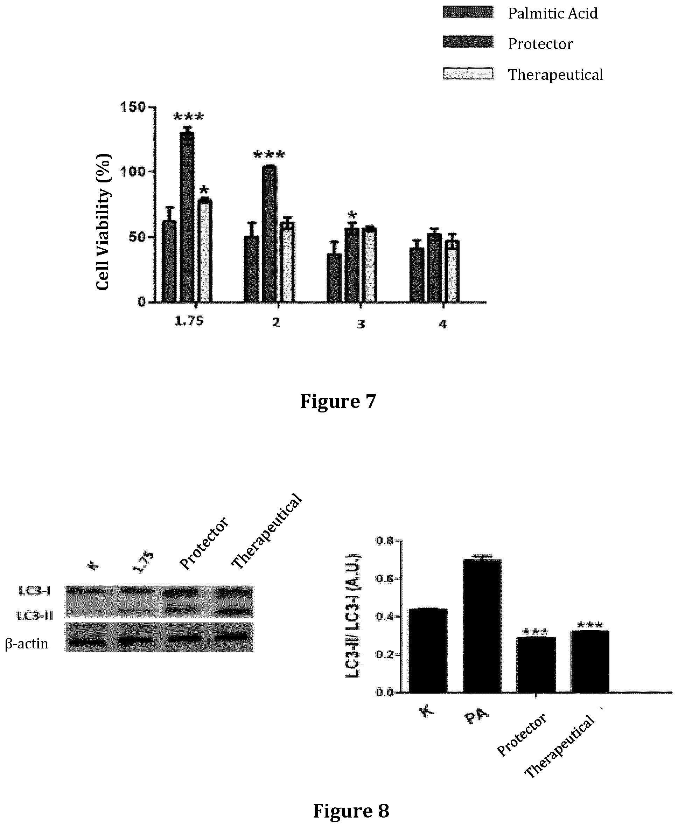

Prior to the application of palmitic acid doses of 1.75, 2, 3 and 4 mM selected as autophagic, apoptotic and necrotic on INS-1 beta cells, in order to observe protective effect of curaglutide on cell death, cells were treated with 10 nM curaglutide for 24 hours. Afterwards curaglutide was removed from the medium, palmitic acid together with 10 nM curaglutide was supplied to a final volume of 5 ml which was kept for 24 hours. Afterwards, in order to observe curaglutide's therapeutic effect, 1,75, 2, 3 and 4 mM of palmitic acid were applied for 24 hours, and cells were treated with 10 nM curaglutide for 24 hours. Both the therapeutic and protective effect of Curaglutide on cell death is demonstrated in Figure 7.

According to the results, % cell viability values were found as; $60.6 \pm 11.6$ in the 1.75 mM (autophagic dose) palmitic acid group, $129.9 \pm 4.6$ in the group where 10 nM curaglutide was administered as a protector against autophagic dose, $78.3 \pm 1.6$ in the group where 10 nM curaglutide was administered as a curative agent for autophagic dose, $50.1 \pm 11$ in the group where 2 mM (apoptotic dose) palmitic acid was administered, $103.9 \pm 0.6$ in the group where 10 nM curaglutide was administered as a protector against apoptotic dose, $60.9 \pm 4.3$ in the group where 10 nM curaglutide was administered as a curative agent for the apoptotic dose, $36.50 \pm 3.3$ in the group where 3 mM (necrotic dose) palmitic acid was administered, $56.4 \pm 4.7$ in the group where 10 nM curaglutide was administered as a protective against necrotic dose, $56.5 \pm 1.7$ in the group where 10 nM curaglutide was administered as a curative agent for the necrotic dose, $41.04 \pm 6.6$ in the group where 4 mM (necrotic dose) palmitic acid was administered, $52.2 \pm 4.6$ in the group where 10 nM curaglutide was administered as a protector against 4 mM of palmitic acid dose, 46.6±5.8 in the group where 10 nM curaglutide was administered as a curative agent for 4 mM palmitic dose. When the groups were compared with each other, a statistically meaningful result among doses was achieved (p<0,05; p<0,0001).

Protective effect of 10 nM curaglutide was shown at 1.75, 2, 3 and 4 mM, wherein its protective effect occurred in a statistically meaningful level at 1,75, 2 and 3 mM (p<0,05; p<0,0001).

In order to observe the therapeutic effect of 10 nM curaglutide, first of all palmitic acid treatment at 1.75, 2, 3 and 4 mM doses were carried out for 24 hours. Afterwards palmitic acid was removed from the medium wherein 10 nM of curaglutide treatment was carried out for 24 hours. Although curaglutide was observed to be therapeutic at doses of 1.75, 2, 3 and 4 mM, therapeutic effect occurred in a statistically meaningful level only at 1,75 mM (p<0,05).

B) Protective and Therapeutic Effect of Curaglutide on Autophagy

Intracellular LC3 protein amount was observed by western blotting in order to examine the effect of curaglutide on autophagy stimulated by 1,75 mM palmitic acid in INS-1 beta cells. Both western blot bands and graph obtained by the calculation according to analysis of the bands were shown in Figure 8.

According to the results, the amount of intracellular LC3-II was found 0.44±0.00 in the control group, 0.70±0.01 in the group where 1.75 mM (autophagic dose) dose of palmitic acid was administered, 0.29±0.00 in the group where curaglutide was administered as protector in the autophagic dose, 0.32±0.00 in the group where curaglutide was administered as a curative agent in the autophagic dose. As a result of paired comparisons, itwas detected that 10 nM of curaglutide generated protective and therapeutic effect in autophagy and these effects were occurring in a statistically meaningful level (p<0,0001).

C) Protective and Therapeutic Effect of Curaglutide on Apoptosis

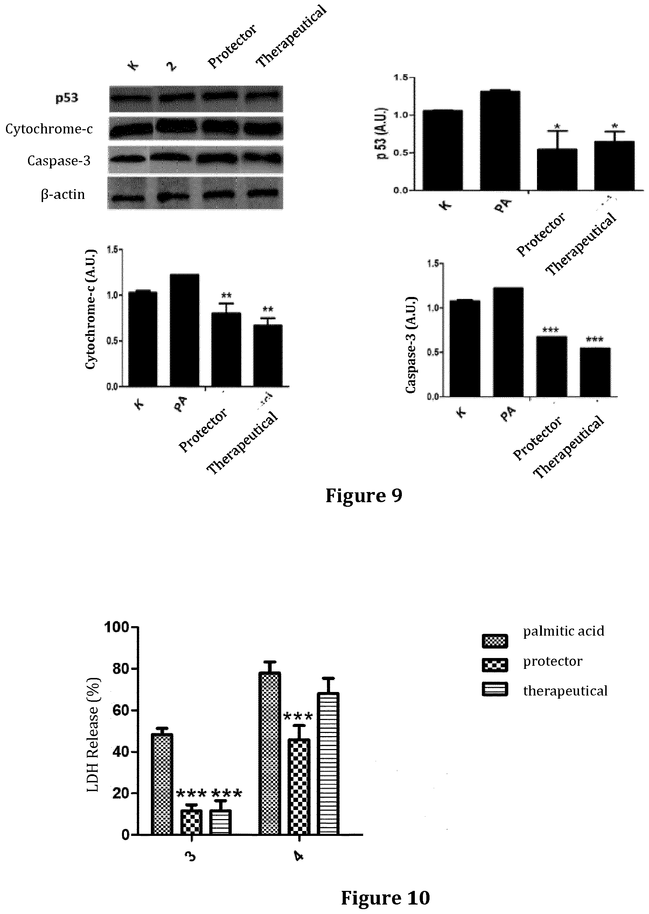

The amount of intracellular proteins such as p53, cytochrome-c and active caspase-3 were observed by western blotting in order to examine the effect of curaglutide on apoptosis stimulated by 2 mM palmitic acid in INS-1 beta cells. Both western blot bands and graph obtained by the calculation according to analysis of the bands are shown in Figure 9.

According to the results, the amount of intracellular p53 was found as 1.06±0.00 in the control group, 1.31±0.02 in the group where 2 mM (apoptotic dose) of palmitic acid was administered, 0.54±0.24 in the group where 10 nM curaglutide was administered as protector against the apoptotic dose, 0.65±0.13 in the group where 10 nM curaglutide was administered as a curative agent in the apoptotic dose. According to paired comparison results, it was detected that the amount of p53 protein had decreased in protective and therapeutic groups as compared to the group where palmitic acid was administered, and this decrease occurred in a statistically meaningful level (p<0,05).

The amount of cytochrome-c was found as 1.02±0.02 in the control group, 1.22±0.00 in the group where 2 mM (apoptotic dose) of palmitic acid was administered, 0.80±0.10 in the group where 10 nM curaglutide was administered as protector against the apoptotic dose, 0.67±0.07 in the group where 10 nM curaglutide was supplied as a curative agent in the apoptotic dose. According to the paired comparison results, it was detected that the amount of cytochrome-c protein was decreased in protective and therapeutic groups as compared to the group where palmitic acid supplied, and this decrease occurred in a statistically meaningful level (p<0,001).

The amount of active caspase-3 was found as 1.07±0.01 in the control group, 1.22±0.00 in the group where 2 mM (apoptotic dose) of palmitic acid was administered, 0.67±0.00 in the group where 10 nM curaglutide was administered as a protector against the apoptotic dose, 0.54±0.00 in the group where 10 nM curaglutide was administered as a curative agent in the apoptotic dose. According to the paired comparison results, it was detected that the amount of active caspase-3 protein was decreased in protective and therapeutic groups as compared to the group where palmitic acid administered, and this decrease occurred in a statistically meaningful level (p<0,0001)

D) Protective and Therapeutic Effect of Curaglutide on Necrosis

Protective and therapeutic effect of 10 nM curaglutide on necrosis is shown in Figure 10 by the evaluation of LDH secretion at 3 and 4 mM doses in INS-1 beta cells. According to the results, in INS-1 beta cells % cytotoxicity values are 48.2±2 in the group where 3 mM dose (necrotic dose) of palmitic acid was administered, 11.6±2.9 in the group where 10 nM curaglutide was administered as a protective against necrotic dose, 11.6±4.8 in the group where 10 nM curaglutide was supplied as a curative agent in necrotic dose, 81.3±5.9 in the group where 4 mM dose of palmitic acid was administered, 45.8±6.8 nM in the group where curaglutide was administered as a protective against 4 mM palmitic acid, 68.2±7.2 in the group where 10 nM curaglutide was administered as therapeutic at 4 mM dose of palmitic acid. As compared to the control group, statistically meaningful results were obtained in both doses in terms of protective effect (p<0,0001). However, curaglutide generates only protective effect in the group where 4 mM palmitic acid was administered. As a result of paired comparisons, it was detected that curaglutide generates both therapeutic and protective effects in the necrotic dose and these effects occurred in a statistically meaningful level (p<0,0001).

**Example 17: The Effect of Curaglutide on the Production of Reactive Oxygen Species in INS-1 Beta Cells in Lipotoxic Conditions**

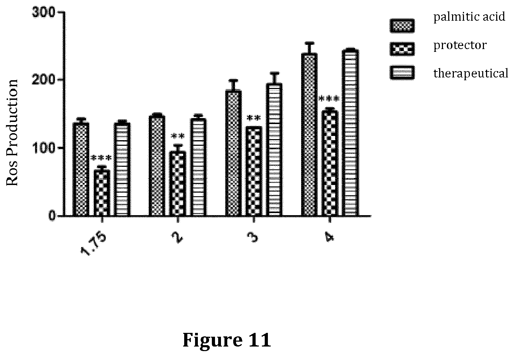

ROS measurement was performed by applying 10 nM curaglutide as protector and therapeutical to INS-1 beta cells wherein palmitic acid was applied in different doses, and the results are shown in Figure 11.

According to the results, ROS measurement percentages in INS-1 beta cells were found as $136.5 \pm 5.6\%$ in the group wherein 1.75 mM palmitic acid was administered, $65.8 \pm 6.2\%$ in the group where 10 nM curaglutide was administered to the autophagic (1.75 mM) dose as protector, $135.3 \pm 4.5\%$ in the group where 10 nM curaglutide was administered to the autophagic (1.75 mM) dose as a curative agent; $145.8 \pm 3.8\%$ in the group where 2 mM palmitic acid was administered, $141.6 \pm 6.1\%$ in the group where 10 nM curaglutide was administered to the apoptotic (2 mM) dose as a curative agent; $174.8 \pm 9.9\%$ in the group where 3 mM palmitic acid was administered, $130 \pm 0.4\%$ in the group where 10 nM curaglutide was administered to the necrotic (3 mM) dose as a protector, $193 \pm 4.4\%$ in the group where 10 nM curaglutide was administered to the necrotic (3 mM) dose as a curative agent; $262.2 \pm 9.9\%$ in the group where 4 mM palmitic acid was administered, $153 \pm 5.0\%$ in the group where 10 nM curaglutide was administered to the necrotic (4 mM) dose as a protector, $242 \pm 2.7\%$ in the group where 10 nM curaglutide was administered to the necrotic (4 mM) dose as a curative agent. According to the binary comparisons, it was determined that curaglutide provides statistically meaningful protecting effect in the cells wherein 1.75, 2, 3 and 4 mM palmitic acid was administered (p<0,001; p<0,0001).

To summarize the experimental study and its results given above;

- It was shown that curaglutide which is a novel GLP-1 analogue increases the viability of lipotoxicity formed INS-1 beta cells.
- It was shown that curaglutide has both therapeutical and protective effect on autophagy, apoptosis and necrosis stimulated by palmitic acid in INS-1 beta cells.
- As a result of all of these studies performed, it was shown that curaglutide which is assessed for the first time for the stated purposes, has antiapoptotic, antiautophagic and antinecrotic properties.

These results obtained as a result of the study show that the invention effectively solves the technical problems purported to be solved herein.

## Claims

1. A pharmaceutical agent comprising Curaglutide alone or in combination with further active agents for use in the prophylaxis and/or treatment of pre-diabetes, diabetes, obesity or metabolic diseases associated thereto.

2. A pharmaceutical agent for use according to claim 1, wherein said pre-diabetes, diabetes, obesity or metabolic diseases associated thereto are the diseases associated with apoptotic, autophagic or necrotic cell death caused by lipotoxicity, glucotoxicity or glucolipotoxicity.

3. A pharmaceutical agent for use according to claim 1, wherein said diabetes is Type I or Type II diabetes.

4. A pharmaceutical agent for use according to claim 1, wherein said pre-diabetes is **characterized by** high fasting glucose levels or impaired glucose tolerance or development of insulin resistance.

5. A pharmaceutical agent for use according to claim 1, wherein said pre-diabetes, diabetes, obesity or metabolic diseases associated thereto are selected from the group consisting of diabetic retinopathy, diabetic maculopathy, glaucoma, retinopathy, neuropathy, nephropathy, foot ulcers, hypertension, hyperlipidemia, metabolic syndrome, gall bladder diseases, osteoarthritis, cardiovascular diseases, stroke, sleep apnea, hepatopathy, asthma, respiratory disorder, menstruation disorders, musculoskeletal disorders, dermatologic disorders, polycystic ovary syndrome and immune system disorders.

6. A pharmaceutical agent for use according to claim 1, wherein said pharmaceutical agent further comprises at least one additional agent selected from the group consisting of anti-obesity agents, anti-inflammatory agents, antidiabetic agents, insulin analogues, insulin sensitizer agents, insulin secretagogues, aldose reductase inhibitors, alpha glucosidose inhibitors, amylin analogues, peptide analogues, sodium glucose transporter 2 (SGLT) inhibitors, and glucosuric agents.

7. A pharmaceutical agent for use according to claim 6, wherein said antidiabetic agent is selected from the group consisting of insulin, insulin lispro, insulin aspart, insulin glulisine, insulin zinc, isophane insulin, insulin glargine, insulin detemir, metformin, phenformin, buformin, ciglitazone, darlitazone, englitazone, lobeglitazone, netoglitazone, rivoglitazone, aleglitazar, saroglitazar, tesaglitazar, rosiglitazone, pioglitazone, troglitazone, acetohexamide, carbutamide, chlorpropamide, metahexamide, tolbutamide, tolazamide, glibenclamide, glibornuride, glicetanile, gliclazide, gliflumide, glipizide, gliquidone, glisoxepide, glyclopyramide, glimepiride, repaglinide, mitiglinide, exenatide, liraglutide, taspoglutide, albiglutide, lixisenatide, dulaglutide, semaglutide, alogliptin, anagliptin, gemigliptin, linagliptin, omarigliptin, saxagliptin, sitagliptin, teneligliptin, vildagliptin, fasiglifam, nateglinide, epalrestat, fidarestat, ranirestat, tolrestat, zenarestat, miglitol, acarbose, voglibose, pramlintide, canaglifozin, dapagliflozin, empagliflozin, remogliflozin, sergliflozin, tofogliflozin, benfluorex and bromocriptine.

8. A pharmaceutical agent for use according to claim 6, wherein said anti-obesity agent is selected from the group consisting of 4-methyl amphetamine, amfechloral, amfephentorex, amfepramone, aminorex, amphetamine, atomoxetine, benfluorex, benzphetamine, bupropion, cathine, cathinone, chlorphentermine, ciclazindol, clobenzorex, cloforex, clominorex, clotermine, dexfenfluramine, dextroamphetamine, dexmethylphenidate, dexfenfluramine, dextroamphetamine, dexmethylphenidate, difemetorex, dimethylcathinone, diphemetoxidine, ephedrine, ephedra, ethylamphetamine, etolorex, fenbutrazate, fencamfamin, fenethylline, fenfluramine, fenproporex, fludorex, fluminorex, furfenorex, indanorex, khat, levopropylhexedrine, lisdexamfetamine, manifaxine, mazindol, mefenorex, methamphetamine, methylphenidate, norfenfluramine, pemoline, pentorex, phendimetrazine, phenethylamine, phenmetrazine, phentermin, phenylpropanolamine, picilorex, pipradrol, prolintane, propylhexedrine, pseudo-efedrine, pyrovalerone, radafaxine, reboxetine, setazindol, sibutramine, sinephrine, tesofensine, viloxazine, xylopropamine, zylofuramine, drinabant, ibipinabant, otenabant, rimonabant, rosonabant, surinabant, taranabant, 5-HTP, galactomannan, glucomannan, L-DOPA, L-phenylalanine, L-tryptophan, L-tyrosine, lorcaserin, Lu AA-33810, naltrexone, naloxone, oxyntomodulin, P57, peptide YY, topiramate, yohimbine, zonisamide, cetilistat, 2,4-dinitrophenol, dirlotapide, mitratapide, oleoyl estrone, orlistat, simmondsin and sterculia.

9. A pharmaceutical agent for use according to claim 6, wherein said anti-inflammatory agent is selected from the group consisting of pyrazolone/pyrazolidines, salicylates, acetic acid derivatives, oxicams, propionic acid derivatives, N-aryl anthranilic acids and coxibs.

10. A pharmaceutical agent for use according to claim 9, wherein said anti-inflammatory agent is selected from the group consisting of aminophenazone, ampyrone, clofenazon, famprofazone, feprazone, kebuzone, metamizole, mofebutazone, morazone, nifenazone, oxifenbutazone, phenazone, phenylbutazone, propyphenazone, sulfinpyrazone, suxibuzone, acetylsalicylic acid, aloxiprin, benorylate, carbasalate calcium, diflunisal, ethenzamide, guacetisal, magnesium salicylate, methyl salicylate, salsalate, salicylamide, salicyclic acid, sodium salicylate, aceclofenac, acemetacin, alclofenac, amphenac, bendazac, bromfenac, bumadizone, bufexamac, diclofenac, difenpramide, etodolac, felbinac, fenclozic acid, fentiazac, indometacin, indometacin farnesyl, isoxepac, ketorolac, lonazolac, acemetacin, prodolic acid, proglumetacin, sulindac, tiopinac, tolmetin, zomepirac, ampiroxicam, droxicam, lornoxicam, meloxicam, piroxicam, tenoxicam, alminoprofen, benoxaprofen, carprofen, dexibuprofen, dexketoprofen, fenbufen, fenoprofen, flunoxaprofen, flurbiprofen, ibuprofen, ibuproxam, indoprofen, ketoprofen, loxoprofen, miroprofen, naproxen, oxaprozin, pirprofen, suprofen, tarenflurbil, tepoxalin, tiaprofenic acid, vedaprofen, naproxcinod, azapropazone, etofenamate, flufenamic acid, flunixine, meclofenamic acid, mefenamic acid, morniflumate, niflumic acid, tolfenamic acid, flutiazin, apricoxib, celecoxib, cimicoxib, deracoxib, etoricoxib, firocoxib, lumiracoxib, mavacoxib, parecoxib, robenacoxib, rofecoxib, valdecoxib, aminopropionitrile, benzydamine, chondroitin sulphate, diacerein, fluproquazone, glucosamine, glucosaminoglycan, hyperforin, nabumetone, nimesulide, oxaceprol, proquazone, tenidap, and orgotein.

11. A pharmaceutical agent for use according to claim 1, wherein said pharmaceutical agent further comprises at least an excipient.

12. A pharmaceutical agent for use according to claim 11, wherein said pharmaceutical agent comprises an agent selected from nanoparticles, liposomes, sterile water, physiological saline solution and cell culture mediums of prior art for targeting said pharmaceutical agent to the cells and for cellular transportation of these pharmaceutical agents.

13. A pharmaceutical agent for use according to claim 11, wherein said pharmaceutical agent is in a form which can be administered via oral, rectal, nasal or vaginal route, or in a form suitable for an administration via intratumoral, subcutan, intracutan, intravenous, intracerebroventricular, intramuscular, intra-arterial, intratracheal, intraperitoneal, parenteral, or topical routes.

14. A pharmaceutical agent for use according to claim 13, wherein said pharmaceutical agent is in an injectable form.

15. A pharmaceutical agent for use according to any of the preceding claims, wherein said pharmaceutical agent has a dose ranging from 0.0001 $\mu$g/kg to 500 mg/kg.

16. An in vitro method for the proliferation of pancreatic beta cells comprising treatment of the beta cells with curaglutide.

**Figure 1**

**Figure 2**

**Figure 3**

**Figure 4**

**Figure 5**

**Figure 6**

Figure 7

Figure 8

**Figure 9**

**Figure 10**

**Figure 11**

Europäisches
Patentamt

European
Patent Office

Office européen
des brevets

**EUROPEAN SEARCH REPORT**

Application Number

EP 17 21 1036

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 012 188 B1 (PHARIS BIOTEC GMBH [DE]) 18 August 2004 (2004-08-18) | 1-6, 11-15 | INV. A61K38/22 A61P3/10 A61K45/06 |
| Y | * paragraphs [0005] - [0008], [0017] * | 1-16 | |
| X | WO 2013/060887 A1 (PHARIS BIOTEC GMBH [DE]) 2 May 2013 (2013-05-02) * pages 1-2; claims * | 1,2,5, 11-15 | |
| X | EP 2 630 965 A1 (CURATIS PHARMA GMBH [DE]; HANNOVER MED HOCHSCHULE [DE]) 28 August 2013 (2013-08-28) * paragraphs [0009] - [0013]; claims * | 1,2, 11-15 | |
| X | EP 1 886 692 A1 (ASUBIO PHARMA CO LTD [JP]) 13 February 2008 (2008-02-13) | 1-5, 11-15 | |
| Y | * paragraphs [0017] - [0020], [0043]; claims * | 1-16 | |
| X | WO 2011/123943 A1 (MOUNT SINAI HOSPITAL CORP [CA]; DRUCKER DANIEL J [CA]; BAGGIO LAURIE L) 13 October 2011 (2011-10-13) * page 22, line 17; claims * | 1,2, 11-15 | TECHNICAL FIELDS SEARCHED (IPC)  A61K |
| X | WO 2004/005342 A1 (ZEALAND PHARMA AS [DK]; STEINESS EVA [DK]) 15 January 2004 (2004-01-15) | 1-5, 11-15 | |
| Y | * pages 10-11; claims * | 1-16 | |
| Y | DE 199 21 537 A1 (HOERSCH DIETER [DE]) 23 November 2000 (2000-11-23) * claims * | 1-16 | |

-/--

The present search report has been drawn up for all claims

| Place of search | Date of completion of the search | Examiner |
|---|---|---|
| Munich | 17 May 2018 | Steendijk, Martin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 1 of 3

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YOUNG-SUN LEE ET AL: "Anti-diabetic actions of glucagon-like peptide-1 on pancreatic beta-cells", METABOLISM, CLINICAL AND EXPERIMENTAL., vol. 63, no. 1, 1 January 2014 (2014-01-01), pages 9-19, XP55475627, US ISSN: 0026-0495, DOI: 10.1016/j.metabol.2013.09.010 * the whole document * | 1-16 | |
| Y | QIAN WEI ET AL: "Exendin-4, a glucagon-like peptide-1 receptor agonist, inhibits cell apoptosis induced by lipotoxicity in pancreatic [beta]-cell line", PEPTIDES, vol. 37, no. 1, 1 September 2012 (2012-09-01), pages 18-24, XP55475576, AMSTERDAM, NL ISSN: 0196-9781, DOI: 10.1016/j.peptides.2012.06.018 * abstract * | 1-16 | TECHNICAL FIELDS SEARCHED (IPC) |
| A | CRISTINA HERN?NDEZ ET AL: "Topical administration of GLP-1 receptor agonists prevents retinal neurodegeneration in experimental diabetes", DIABETES, 17 September 2015 (2015-09-17), page db150443, XP55422205, US ISSN: 0012-1797, DOI: 10.2337/db15-0443 * abstract * | 1-16 | |

-/--

The present search report has been drawn up for all claims

| Place of search | Date of completion of the search | Examiner |
|---|---|---|
| Munich | 17 May 2018 | Steendijk, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches
Patentamt

European
Patent Office

Office européen
des brevets

## EUROPEAN SEARCH REPORT

Application Number

EP 17 21 1036

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHEN SONG ET AL: "Inhibiting receptor for advanced glycation end product (AGE) and oxidative stress involved in the protective effect mediated by glucagon-like peptide-1 receptor on AGE induced neuronal apoptosis", NEUROSCIENCE LETTERS, ELSEVIER, AMSTERDAM, NL, vol. 612, 8 December 2015 (2015-12-08), pages 193-198, XP029392279, ISSN: 0304-3940, DOI: 10.1016/J.NEULET.2015.12.007 * abstract * ----- | 1-16 | |
| | | | TECHNICAL FIELDS SEARCHED (IPC) |

The present search report has been drawn up for all claims

| Place of search | Date of completion of the search | Examiner |
|---|---|---|
| Munich | 17 May 2018 | Steendijk, Martin |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 3 of 3

## ANNEX TO THE EUROPEAN SEARCH REPORT
## ON EUROPEAN PATENT APPLICATION NO.

EP 17 21 1036

This annex lists the patent family members relating to the patent documents cited in the above-mentioned European search report.
The members are as contained in the European Patent Office EDP file on
The European Patent Office is in no way liable for these particulars which are merely given for the purpose of information.

17-05-2018

| Patent document cited in search report | | Publication date | Patent family member(s) | | Publication date |
|---|---|---|---|---|---|
| EP 1012188 | B1 | 18-08-2004 | AT | 273996 T | 15-09-2004 |
| | | | DE | 59811840 D1 | 23-09-2004 |
| | | | EP | 1012188 A1 | 28-06-2000 |
| | | | ES | 2222613 T3 | 01-02-2005 |
| | | | JP | 2001516765 A | 02-10-2001 |
| | | | US | 2005130891 A1 | 16-06-2005 |
| | | | US | 2008242609 A1 | 02-10-2008 |
| | | | US | 2014024585 A1 | 23-01-2014 |
| | | | WO | 9914239 A1 | 25-03-1999 |
| WO 2013060887 | A1 | 02-05-2013 | CN | 104039344 A | 10-09-2014 |
| | | | EP | 2771025 A1 | 03-09-2014 |
| | | | JP | 2014530892 A | 20-11-2014 |
| | | | US | 2016207970 A1 | 21-07-2016 |
| | | | WO | 2013060887 A1 | 02-05-2013 |
| EP 2630965 | A1 | 28-08-2013 | NONE | | |
| EP 1886692 | A1 | 13-02-2008 | AU | 2006250347 A1 | 30-11-2006 |
| | | | BR | PI0610089 A2 | 09-12-2008 |
| | | | CA | 2606902 A1 | 30-11-2006 |
| | | | CN | 101184509 A | 21-05-2008 |
| | | | EP | 1886692 A1 | 13-02-2008 |
| | | | JP | WO2006126688 A1 | 25-12-2008 |
| | | | KR | 20080011675 A | 05-02-2008 |
| | | | US | 2009233851 A1 | 17-09-2009 |
| | | | WO | 2006126688 A1 | 30-11-2006 |
| WO 2011123943 | A1 | 13-10-2011 | EP | 2555791 A1 | 13-02-2013 |
| | | | US | 2013053316 A1 | 28-02-2013 |
| | | | WO | 2011123943 A1 | 13-10-2011 |
| WO 2004005342 | A1 | 15-01-2004 | AT | 432289 T | 15-06-2009 |
| | | | AU | 2003243929 A1 | 23-01-2004 |
| | | | AU | 2009202390 A1 | 09-07-2009 |
| | | | AU | 2010257216 A1 | 13-01-2011 |
| | | | CA | 2490564 A1 | 15-01-2004 |
| | | | DK | 1525219 T3 | 07-09-2009 |
| | | | EP | 1525219 A1 | 27-04-2005 |
| | | | EP | 2028192 A1 | 25-02-2009 |
| | | | ES | 2327328 T3 | 28-10-2009 |
| | | | HK | 1075456 A1 | 31-12-2009 |
| | | | IL | 165531 A | 31-01-2011 |
| | | | IL | 208184 A | 31-12-2014 |
| | | | JP | 5685355 B2 | 18-03-2015 |
| | | | JP | 5758870 B2 | 05-08-2015 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**

EP 17 21 1036

This annex lists the patent family members relating to the patent documents cited in the above-mentioned European search report.
The members are as contained in the European Patent Office EDP file on
The European Patent Office is in no way liable for these particulars which are merely given for the purpose of information.

17-05-2018

| Patent document cited in search report | Publication date | Patent family member(s) | | Publication date |
|---|---|---|---|---|
| | | JP | 2006515267 A | 25-05-2006 |
| | | JP | 2013126981 A | 27-06-2013 |
| | | MX | PA04012497 A | 14-07-2005 |
| | | US | 2006057137 A1 | 16-03-2006 |
| | | US | 2009088369 A1 | 02-04-2009 |
| | | US | 2014187483 A1 | 03-07-2014 |
| | | WO | 2004005342 A1 | 15-01-2004 |
| DE 19921537 A1 | 23-11-2000 | NONE | | |

EPO FORM P0459

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6268343 B1
- WO 2005000892 A2
- WO 2007012188 A
- WO 2013060887 A1

**Non-patent literature cited in the description**

- **SALLING, H., K.** *Regulatory peptides,* 2012, vol. 178, 51-55